Europäisches Patentamt

(19) **European Patent Office**

Office européen des brevets

(11) Publication number: **0 032 124**
**A2**

(12) # EUROPEAN PATENT APPLICATION

(21) Application number: **81300009.8**

(22) Date of filing: **02.01.81**

(51) Int. Cl.³: **A 61 K 6/08**
**C 08 J 3/12, C 08 F 265/06**

(30) Priority: **04.01.80 DE 3000213**

(43) Date of publication of application:
**15.07.81 Bulletin 81/28**

(84) Designated Contracting States:
**CH GB IT LI**

(71) Applicant: **DENTSPLY INTERNATIONAL, INC.**
**570 West College Avenue**
**York Pennsylvania 17405(US)**

(72) Inventor: **Komma, Ottmar Hans**
**an den Logesmühle 1**
**D-6361 Nieddtal 4(DE)**

(74) Representative: **Newens, Leonard Eric et al,**
**F.J. CLEVELAND & COMPANY 40/43 Chancery Lane**
**London WC2A 1JQ(GB)**

(54) Process for producing a crosslinked polymerizate for dental purposes.

(57) The invention relates to a process for producing a crosslinked polymerizate which can be employed in the dental field and with high swelling rate by copolymerization of at least one monomer with at least one crosslinking agent with at least two isolated double bonds, characterized in that for increasing the swelling rate, a surface layer of the obtained partially crosslinked polymerizate particles is subjected to a structure disaggregation by incorporating a non-crosslinking monomer and subsequent polymerization thereof and/or by physical action.

EP 0 032 124 A2

Croydon Printing Company Ltd.

COMPLETE DOCUMENT

## DESCRIPTION

The invention relates to a process for producing a crosslinked polymerizate which can be employed in the dental field, as well as to a dental material with a content of this polymerizate, and to the use of this dental material for the fabrication of dental products as in particular teeth, crowns, bridges or veneering material, fillings and denture material.

Dental products, especially artificial teeth, crowns, bridges, and veneering material are increasingly produced of plastic materials. For this purpose, usually two components namely a powdery component of at least one methyl methacrylate bead polymerizate and a liquid component of methyl methacrylate and crosslinking monomers with several double bonds are used. For the fabrication of a dental product, these two components are spatulated, the liquid component partly penetrating into the polymer particles, so that a plastic, well moldable mass is produced. This material is then molded to artificial teeth, crowns, bridges or the like and subsequently polymerized. Polymerization is either by irradiation or thermal decomposition of a catalyst previously incorporated into the powder component I. This way a dental product with a matrix of crosslinked methyl methacrylate polymerizate, in which uncrosslinked particles of methyl methacrylate polymerizate are embedded, is produced. The dental material used in this process is distinguished by good handling properties. In particular, an uncrosslinked methyl methacrylate polymerizate shows an excellent swelling behavior in the liquid component so that a useful moldable plastic mass is obtained already after a short spatulation period.

By the use of mixtures of different polymerizates, a long pot life can be obtained in an easy way. Beyond that, the dental products obtained have a low brittleness. They do, however, show a number of critical disadvantages. On the one hand, the finishing of the modelled and cured dental products, in particular the grinding and polishing, involves difficulties. The grinding hardness is low and in consequence of the frictional heat, softening of the plastic material is the case, and accordingly deformations and smearing result. Also, the abrasion resistance is insufficient. This is indicated by the formation of grooves caused by the tooth brushes, tooth pastes and the like after the teeth have been worn for a longer period.

For this reason, hard inorganic fillers were introduced into the dental material, e.g., glass powder or quartz powder, in order to increase the abrasion resistance and the grinding hardness. However, at the same time, the polishability is reduced since during polishing work, inorganic filler particles break off the surface layer and lead to the formation of grooves (German OS 23 12 258). It has been recognized that polishability and abrasion resistance have an antagonistical relationship to each other. This applies in particular to filler particle sizes in the range of 1 to 20 $\mu$. To eliminate these difficulties, trials were performed on extremely fine Aerosil (Reports of the Institute for Medical and Dental Engineering, Tokyo Medical and Dental University, 2, 55-61 [1968]). This measure indeed leads to a considerable improvement of polishability, obtaining a high abrasion resistance and high grinding hardness at the same time. The condition, however, is a high content of Aerosil. On the other hand, this results in increased brittleness so that during processing the dental products or during usage, portions of the dental material may break off. This applies in particular to the incisal area. Consequently, it is not possible any more to build up the incisal area free to obtain a transparency. Rather the whole tooth up to the incisal area has to be backed with metal. Reducing the Aerosil content results, naturally, in a lower brittleness; on the other hand, the benefits of a high abrasion resistance and a high grinding hardness get partially lost.

It has already been attempted to realize an increase in abrasion resistance and grinding hardness, avoiding an augmentation of brittleness, by using crosslinked polymerizate particles as powder component (US Patent 3,470,615 and 3,471,596). In this connection, however, substantial impairments of the processibility of the dental material have to be put up with. By the crosslinking of the polymerizate particles, the gelling ability within the monomers used is reduced so that the short spatulation periods, requested in practice, until a useful mass is obtained (approximately 2 min) are not achieved. Rather the crosslinked polymerizate particles - if still gelable at all - require extremely long gelling times. The crosslinked polymerizate particles are, therefore, present in the paste as dead material so that the former does not have a plasticity and involves difficulties when modelling. A certain remedy is possible by the addition of non-crosslinked polymethyl methacrylate since it gels easily and increases the ability to prepare the dough and to model it. This, however, again leads to a reduced abrasion resistance.

It is, therefore, the task of the present invention to develop a dental material which on the one hand leads to dental products with high abrasion resistance, excellent polishability, high grinding hardness, and extremely low brittleness, and on the other hand, produces a plastic, easily moldable paste at a short gelling time, with a long pot life, and in addition where the modelled, cured dental products do not tend to softening and smearing when grinding and polishing.

This task is solved by the invention that for the fabrication of the dental products, a powder component with a partially crosslinked polymerizate is used which was subjected to a modification treatment increasing the ability to swell.

This invention, therefore, creates a process for the fabrication of a powdery, partially crosslinked and yet easily gelable polymerizate for dental purposes which is characterized by the main claim. In addition, according to the claims, dental materials with a content of this modified, crosslinked polymerizate component are created as well as dental products using these dental materials.

First, the production of a partially crosslinked polymerizate for dental purposes, to be subjected to the subsequent modification treatment shall be described.

As is known, monomers with only one double bond, e.g. methylmethacrylate, lead to uncrosslinked linear polymers whilst monomers with several double bonds, e.g. allylmethacrylate or ethylene glycol dimethacrylate, lead to a three-dimensional polymer frame with space crosslinked structure. Using a mixture of monomers with a double bond and monomers with several double bonds, a "partially crosslinked" polymerizate is obtained. According to the invention, such a partially crosslinked polymerizate is preferred.

Monomers with only one double bond are called "monomers" below. Monomers with several isolated double bonds are below called "crosslinking agents". For the preparation of a partially crosslinked polymerizate, all conventional monomers suitable for the dental field are adequate, in particular acrylic esters and lower alkyl acrylic esters, N-vinyl lactams, acrylamides, acrylonitriles, styrenes, alkenes, and urethanes. Mixtures of two or several different monomers may be employed.

Preferred are esters of acrylic acid or of an $\alpha$-$C_{1-6}$-alkyl acrylic acid, e.g. of methacrylic acid of $\alpha$-ethyl acrylic acid or of $\alpha$-propyl acrylic acid and of an aliphatic or aromatic alcohol or phenol. Preferred are aliphatic and cycb-aliphatic alcohols with 1 to 20 and preferably 1 to 6 carbon atoms or also phenol and $C_{1-6}$-alkyl phenol. The alkyl of the acid or the alcohol of the ester can have one or several substituents, in particular hydroxyl groups, amino groups, thiolic groups, and halogen atoms (fluorine, chlorine, bromine, iodine).

Examples of preferred monomers are: Methyl-, ethyl-, isopropyl-, tert-butyl-, octyl-, dodecyl-, cycbhexyl-, chloromethyl-, tetrachloro-ethyl-, perfluorooctyl-, hydroxyethyl-, hydroxypropyl-, hydroxybutyl-, 3-hydroxyphenyl-, 4-hydroxyphenyl-, aminoethyl-, aminophenyl-, thiophenyl-, tetrahydrofurfuryl-, tetrahydropyranyl-acrylate or -methacrylate, -ethacrylate, -propacrylate, -butacrylate or -chloromethacrylate. Furthermore, monoacrylic esters or monalkyl acrylic esters of bisphenol A, dihydroxydiphenyl sulfone, dihydroxydiphenyl ether, dihydroxydiphenyl, dihydroxydiphenyl sulfoxide and 2,2-bis-(4-hydroxy)-2,3,5,6-tetrafluorophenyl propane are appropriate. It is also possible to use mixtures of two or more than two different acrylates or alkyl acrylates. Specially preferred is methyl methacrylate.

With the acrylate or alkyl acrylate, other co-monomers may be used additionally, i.e. in particular the monomers indicated earlier. Preferred co-monomers are N-vinyl caprolactame, acrylic acid amide, methacrylic acid amide, acrylo-nitrile, methacrylic

nitrile, styrene, α-methyl styrene, ethylene,                    propylene,
butanes, pentanes, hexanes or urethane acrylates or -methacrylates,
vinyl esters as vinyl acetate or vinyl propionate, dienes as
butadiene, isoprene, dimethyl butadiene or chloroprene, vinyl
ethers as ethyl vinyl ether or β-ethyl chloride vinyl ether.  Up
to 40% and preferably up to 30% of the acrylate or alkyl acrylate
are preferably substituted by the comonomer.

As "crosslinking agents" a number of different monomers
with two or several crosslinking functional groups are suitable.
Preferred are compounds of two or several isolated ethylenic
double bonds.

For example, suitable are esters of polyhydric alcohols
or phenols with acrylic acid or lower alkyl acrylic acid or
esters of acrylic acid or an alkyl acrylic acid with an alcohol
which in addition has a functional group that acts as crosslinker,
e.g. a double bond; urethane diacrylates and dimethacrylates,
polyvinyl compounds, aromatic divinyl compounds or the like.

As crosslinking agents, preferred are esters of
unsaturated acids, e.g. of acrylic acid or an alkyl acrylic acid
as methacrylic acid, ethacrylic acid, propacrylic acid, butacrylic
acid or the like or esters of maleic acid, fumaric acid, citraconic
acid, mesaconic acid, itaconic acid, aconitic acid or the like.
These esters are esterified preferably with an unsaturated alcohol,
which preferably may be monohydric, or with a polyhydric alcohol.
The alcohol component comprises preferably 2 to 30 and specifically
2 to 20 carbon atoms.  Suitable alcohols are allyl, methallyl,
crotyl, vinyl, butenyl, isobutenyl alcohols as well as polyols
such as ethylene glycol, propylene glycol, butylene glycol, diethylene glycol, triethylene glycol, tetraethylene glycol, pentaethylene glycol, glycerine, 1,3,3-trimethylolpropane, pentaerythrite, dihydroxyphenol, and alkylidene bisphenols such as bisphenol-A, 1,1-bis(4-hydroxyphenyl) methane, 4,4'-dihydroxydiphenyl sulfone, dihydroxydiphenyl ether, dihydroxydiphenyl sulfoxide, resorcinol or bis glycidyl ether or bis-alkylene oxide ether of the polyhydric alcohols and phenols indicated.

Preferred crosslinking agents are allyl acrylate, allyl methacrylate, vinyl acrylate, vinyl methacrylate, dimethallyl fumarate, N-allyl acrylamide, crotyl acrylate, allyl crotonate, allyl cinnamate, diallyl maleate; ethylene glycol diacrylate or dimethacrylate, trimethylolpropane trimethacrylate as well as the bisacrylate or the bismethacrylate of bisphenol-A as well as bisacrylates or bismethacrylates of polyalkylene glycols in particular of polyethylene glycol with a degree of polymerization of up to about 10; bis-acrylic ester and bis-methacrylic ester of bisphenol-A, 4,4'-dihydroxydiphenyl, bis(4-hydroxyphenyl) alkane, 4,4'-dihydroxydiphenyl sulfone, 4,4'-dihydroxydiphenyl sulfoxide, 4,4'-dihydroxydiphenyl ether or 4,4'-dihydroxydiphenylsulfide or the diglycidyl, dialkylene oxide or diurethane derivates thereof; glycidyl acrylate; divinyl benzene, substituted divinyl benzenes or reaction products of hydroxyethyl methacrylate with 2,2,4-trimethylhexyl-1,6-diisocyanate.

The weight ratio of the monomer(s) to the crosslinking agent is preferably within the range of 99.9 : 0.1 - 10 : 90 and in particular within the range of 99 : 1 - 50 : 50 and specifically within the range of 98 : 2 up to 75 : 25. Though not preferred, inorganic fillers, e.g. glass, silicate, quartz, titanium dioxide or the like, and in particular fine-particle fillers, as Aerosil, may also be added to the mixture of crosslinking agents and monomers.

Polymerization may be carried out as photo polymerization. Preferred, however, is a polymerization with a radical initiator, e.g. a peroxide as benzoylperoxide or dicumylperoxide or azobisisobutyronitrile. Polymerization may be effected as suspension polymerization (described in the U.S. Patent 2,673,194) or as emulsion polymerization, solution polymerization or as substance polymerization. Suspension or emulsion polymerization is preferred since a bead polymerizate with the desired particle size is the result. In the case of the substance polymerization, the polymerizate has to be crushed so a splinter polymerizate is obtained. The particle size of the polymerizate obtained is preferably about 0.001 to about 500 μ on an average, where preferably at least 50 weight % of the particles have a diameter below 150μ and specifically below about 100 μ. Specially preferred is a medium particle size of 10 to 50 μ and specifically of 20 to 40 μ.

The following describes the modification treatment of the partially crosslinked polymerizate for the purpose of increasing the ability to swell. Because of the partially crosslinked structure, the molecules of a liquid monomer penetrate the polymer frame only slowly. The swelling of a partially crosslinked methyl methacrylate polymerizate with monomeric methyl methacrylate requires therefore many hours. The inventive modification treatment reduces the swelling time to a few minutes only. The principle of this treatment is to disaggregate or break the crosslinked polymerizate structure within a surface layer and to increase, thus, the penetration ability of the monomer molecules. This can be done the chemical of physical way.

With a chemical method to increase the swellability of the polymerizate particles, a monomer is allowed to react on the polymerizate particles for a longer period so that the monomer can penetrate into the surface layer. Subsequently the monomer is polymerized so that within the surface layer of the partially crosslinked polymerizate particle there develop zones of an uncrosslinked polymerizate. These zones have a high swelling or dissolving capability within the liquid monomer mixture used to prepare the dough. They, therefore, act as penetration tracts for the monomers.

As monomers for the modifying treatment, the monomers mentioned in connection with the preparation of the partially crosslinked polymer are suitable. Again, various different monomers may be employed. Preferred is methyl methacrylate. Crosslinking monomers are not present. The weight ratio of the monomer to the partially crosslinked polymerizate is dependent upon the degree of crosslinking of the polymer, the type of the monomer, and the reaction duration. Preferably, this weight ratio is 1:10 up to 5:1 and especially 1:3 up to 1:1. Preferably exactly that monomer quantity is employed which is to be incorporated into the partially crosslinked polymerizate for obtaining the desired ability to swell. It is also possible to employ an excess monomer quantity and separate it after the desired reaction time has elapsed. The reaction duration is dependent upon the degree of crosslinking of the polymerizate as well as the particle size of the polymerizate and the reaction temperature, the viscosity of the monomer, and the type of the monomer. With a high degree of crosslinking respectively large particle size respectively low temperature respectively high viscosity of the monomer, the reaction period has to be long. With a low degree of crosslinking, small particle size, high temperature, and low viscosity, a short reaction time is chosen. The reaction time may be within the range of 5 minutes to 150 days, especially 30 minutes to 30 days,

0032124

and preferably within the range of 6 to 24 hours. At this stage of the modification treatment, the particles should not become sticky so that agglomeration is prevented. It must be possible to slurry the particles. The subsequent polymerization of the monomer which penetrated into the surface layer of the partially crosslinked polymerizate particles may either be carried out by photopolymerization or thermal decomposition of a compound forming free radicals, which was already mentioned in the description of how to prepare the partially crosslinked polymerizate. This polymerization initiator is added to the monomers or the monomer mix with which the partially crosslinked polymer is treated. Polymerization is under the usual conditions. For this purpose, the monomer-moist particles are slurried in water and heated under pressure. In this process, the polymerization conditions indicated earlier may be employed.

The modification treatment of the surface layer of the partially crosslinked polymerizate particles in terms of a disaggregation and increasing the penetration capability of the monomer molecules, is also possible the physical way. Preferred is in particular a modifying treatment with high energy rays or a mechanical treatment.

With the high energy radiation of polymethacrylate, individual bonds of the polymer chains are disassociated. Therefore, the energy of the ray corpuscles respectively the ray quanta must exceed the chemical linkage energy of the linkage to be disassociated considerably. Appropriate are X-rays, $\gamma$-rays, neutron rays, electron rays, $\alpha$-rays and especially energized consequent nuclei from nuclear reactions with slow neutrons. Preferred are rays with low penetration depth, e.g. with a penetration depth of $1\mu$ up to $1000\mu$ and preferably $10\mu$ up to $100\mu$

in water. The radiation case should be kept in motion during radiation. The radiation dose is preferably $10^2$ up to $10^8$ rad and specifically $10^3$ up to $10^7$ rad.

A further important physical method to modify a surface layer of the partially crosslinked polymerizate particles in the sense of increasing the ability to swell in combination with the monomer concerns the mechanical treatment of the particles. All mechanical treatments causing conditions of stress in the surface layer of the polymerizate particles without however reducing the size of the polymerizate particles appreciably, are suitable. Especially preferred is the treatment of the polymerizate particles in a ball mill. The latter may contain steel balls or ceramic balls. Also suitable are jet impact treatments, high speed stirrers, vibratory mills, roller mills with an opening which is smaller than the particle size, edge mills, disk attrition mills, cutting mills. On all of these mechanical treatments, the surface layer developes fine fissures and/or conditions of deformation. With the subsequent treatment with the monomer, the latter penetrates into the partially crosslinked polymer particle along the fissures or along the areas under stress. The gelling of the polymerizate particles with the monomer may be interpreted as corrosion phenomenon. Through conditions of stress within the surface layer, the corroding is increased. The time period of this mechanical treatment is dependent upon the intensity of the treatment.

When using a ball mill, the period of treatment should be preferably within the range of 1 to 24 hours and specifically within the range of 2 to 12 hours. On the other types of treatment, an equivalent time of treatment is selected. In general, the mechanical treatment should be discontinued as soon as the desired gelability has been obtained. This can be determined by a simple test. On this test, the final composition of partially crosslinked polymerizate modified by mechanical treatment and liquid monomer mix is processed to a dough. Mechanical treatment is adequate as soon as a ready for use mass which is sufficiently plastic through swelling is obtained within a spatulation time of preferably up to 5 minutes and specifically up to 2 minutes.

The partially crosslinked polymerizate modified according to the invention, is used to prepare the powder component of a dental material. For this purpose, the partially crosslinked, modified polymerizate may be combined with an uncrosslinked polymerizate. For the preparation of the uncrosslinked polymerizate, the monomers and comonomers mentioned already in connection with the preparation of the solid polymerizate may be used. Especially polymerizates of esters indicated of acrylic acid and the α-alkyl acrylic acids are suitable, i.e. both homopolymerizates thereof and copolymerizates with other monomers with specially up to 20% and specifically up to 15 and more especialy up to 10% of another monomer. Copolymerizates of methyl methacrylate, and styrene are specially preferred. The uncrosslinked polymerizate should have a particle size within the range of preferably 5 to 500μ and specifically 30 to 40μ. It is added to increase the workability, especially the pot life. The proportion of the modified, partially crosslinked polymerizate toward the uncrosslinked polymerizate is preferably within the range of 10 : 90 up to 95 : 5 and especially within the range of 40 : 60 up to 80 : 20. This uncrosslinked polymerizate is prepared the conventional way, e.g. under the polymerization conditions mentioned earlier.

Part of the uncrosslinked polymerizate with relatively large particle size may be substituted by an uncrosslinked polymerizate with very small particle size in particular with a particle size lower than 1 μ. For this fine polymerizate, the same monomers and comonomers are appropriate. It is obtained by solution polymerization and subsequent spray drying. Preferred is finely particulate polymethyl methacrylate. Preferably up to 50% and especially up to 40% and in particular up to 30% of the coarse uncrosslinked polymerizate may be substituted by a finely particulate polymerizate.

The uncrosslinked polymerizates should have an average molecular weight (determined by viscosity measuring) within the range of about 100,000 up to about 2 million and specifically within the range of about 500,000 up to about 900,000.

The powder component of the dental material may contain several different, modified, partially crosslinked polymerizates in order to improve the workability. Furthermore, an initiator or activator or a component of a common redox system may be added to the powder component. In addition, customarily, pigments, bond modifiers, and the like are added. The addition of an inorganic filler may be advantageously discontinued according to the invention, without impairing the vital properties. It is, however, possible to add fillers. The above mentioned fillers are suitable. The filler content may be preferably up to 50% and specifically up to 30% and in particular up to 20% of the total weight of the powder component. With a high filler content, the physical properties of the dental material and the resultant dental product will be changed. Finely particulate fillers of a particle size below 700 μ, e.g. Aerosil, are specially preferred. The resultant component has an excellent shelf life. Its physical properties and handling characteristics practically hardly change during the storage period.

For the preparation of the liquid component of the dental material, one or several of the above liquid monomers are mixed with one or several of the above crosslinking agents as well as with a stabilizer. The proportion of the monomers to the crosslinking agent is preferably 5:95 up to 99:1 specifically 5:50 up to 90:10. As monomers, again preferably the esters of the acrylic acid and the alkyl acrylic acids mentioned are in question. Especially preferred is methyl methacrylate and/or tetrahydrofuryl methacrylate. As crosslinking agents, preferably di- or polymethacrylates are in question, e.g. polyalkylene glycol dimethacrylate, such as ethylene glycol dimethacrylate, propylene glycol dimethacrylate, diethylene glycol dimethacrylate, dipropylene glycol dimethacrylate, triethylene glycol dimethacrylate, tetraethylene glycol dimethacrylate. On principle, all liquid monomer mixes used so far are suitable. An accelerator, e.g. an amine, may also be added or in the case of an autopolymerization, a customary redox partner. The two components can be processed to dental products the customary way, e.g. crowns, bridges, veneering material, fillings, denture material, and artificial teeth. When mixing the powder component with the liquid component to form a dough, the resultant mass is ready for use after about 2 minutes as the liquid component can penetrate into the modified, partially crosslinked polymerizate particles rapidly. The dental products obtained from the dental material according to the invention disclose a high abrasion resistance, an excellent grinding hardness, and good polishability and beyond that an extremely low brittleness.

The following explains the invention further on the basis of examples.  If not stated otherwise, the data given on proportions and percentages refer to weight.

Example 1

(A)  Preparation of a Partially Crosslinked Polymerizate

200 parts of water, 95 parts of methyl methacrylate, and 5 parts of allyl methacrylate as well as 1,0 part emulsifier and 3 parts benzoyl peroxide are filled into a polymerization vessel.  Subsequently, polymerization is carried out with stirring and under autogenous pressure at a temperature of about 110°C for 3 hours.  Then the reaction mix is cooled and filtered.  The polymerizate obtained is cleaned with water and dried.  It has a medium grain size of 30 up to 40 μ.

(B)  Increasing the Ability to Swell

In one part of methyl methacrylate, 0.01 parts of benzoyl peroxide are dissolved and the resultant solution is mixed with 2 parts of the product of step (A).  At this stage, the mix is left at room temperature for 10 to 12 h when the monomer, together with the benzoyl peroxide, penetrates into the polymerizate particles.  Subsequently, the still monomer-moist product is slurried in 6 parts of water and heated in a pressure vessel to obtain a pressure of 1.8 bar and held at this pressure for 1 h. Under these conditions, the benzoyl peroxide is decomposed and the methyl methacrylate which penetrated into the polymerizate product of step (A) is polymerized.  On cooling, the polymerizate is filtered, cleaned in water, and dried.  It will now evidence an increased swellability in methyl methacrylate.  On the other hand, it is not sticky.

(C)  Preparation of the Polymerizate Mix (Component I)

75 parts of the product of step (B) are mixed with 25 parts of a methyl methacrylate copolymer as well as with pigments (0.8 parts) and benzoylperoxide (1 part).  This mix serves as material for the fabrication of dental products.  It remains serviceable for longer time periods with no esential change in its physical properties and in particular in handling features.

(D)  Preparation of the Liquid Component (Component II)

80 parts of methyl methacrylate and 20 parts of triethylene glycol dimethacrylate as well as 100 ppm hydroquinone are mixed.  The resultant mix can be stored for an unlimited period and will not change.  It serves as material for the fabrication of dental products.

(E)  Fabrication of Dental Products

1 part of the liquid component II, obtained in step (D), is spatulated with 2 parts of the powder component I, obtained in step (C).

The mix obtained is left to gel 2 to 3 minutes from starting to mix.  The resultant composition is perfectly processible after this short swelling time.  The pot life extends to 30 minutes.  During this time, the compound is plastic and well moldable and practically not stringy.  It is then used the usual way for building up a crown or a bridge and the molded case is polymerized in a common polymerization apparatus under pressure. The resulant dental product is then ground as usual, the grinding behavior being excellent with a high grinding resistance.  In particular, no smearing occurs.

When polishing the case subsequently, the excellent polishability of the material is evidence and a dental product is obtained which has an extremely smooth surface, an abrasion resistance of 5 to 6 mg ("Dentallabor" 21, 615-619 [1973]) and with low brittleness.

Example 2

The product of step (A) of example 1 is subjected to the ionizing rays of a customary high energy radiation facility continuously. The radiation dose is $10^5$ rad. This radiation reaction leads to a partial breaking of the structure by which the swelling capability is increased. The steps (C), (D), and (E) of example 1 follow. The compound obtained is also processible after a very short time and shows an excellent processibility. The cured dental product also demonstrates a very good grinding and polishing behavior, a low brittleness, and an increased abrasion resistance.

Example 3

The product of step (A) of example 1 is processed in a ball mill using ceramic balls overnight. In this process, the product is hardly reduced in size and very little abrasion powder is developed. Then steps (C), (D), and (E) of example 1 follow. Again, the same excellent processing properties and product properties as in sample 1 are obtained.

CLAIMS:

1. Method to produce a crosslinked polymerizate with high swelling rate by copolymerization of at least one monomer with at least one crosslinking agent with at least two isolated double bonds, characterized in that for increasing the swelling rate, a surface layer of the obtained partially crosslinked polymerizate particles is subjected to a structure disaggregation by incorporating a non-crosslinking monomer and subsequent polymerization thereof and/or by physical action.

2. Method according to Claim 1, characterized in that as monomer for the production of the crosslinked polymerizate, an ester of acrylic acid or of an alkyl acrylic acid with a monovalent alkyl or cycloalkyl alcohol or a monovalent phenol or alkyl substituted phenol is used.

3. Method according to Claim 2, characterized in that for the production of the partially crosslinked polymerizate, a comonomer in a quantity of up to 40 weight percent is employed.

4. Method according to one of Claims 1 to 3, characterized in that for the production of the partially crosslinked polymerizate, a bis-ester or polyester of a dihydric or polyhydric alcohol or phenol with acrylic acid or alkyl acrylic acid is employed.

5. Method according to one of Claims 1 to 4, characterized in that a weight ratio of monomer to crosslinking agent of 99.9 : 1 to 10:90 is selected.

6. Method according to one of Claims 1 to 5, characterized in that the partially crosslinked polymerizate is produced as bead polymerizate.

0032124

7. Method according to one of Claims 1 to 6, characterized in that in to the partially crosslinked polymerizate particles there is incorporated an ester of acrylic acid or alkyl acrylic acid as a monomer that does not act as a crosslinker.

8. Method according to one of Claims 1 to 7, characterized in that the non-crosslinking monomer is allowed to penetrate into the partially crosslinked polymerizate during a period of 5 minutes to 150 days and preferably 30 minutes to 30 days.

9. Method according to one of Claims 1 to 8, characterized in that a weight ratio of the non-crosslinking monomer to the partially crosslinked polymerizate of 1:10 up to 5:1 is selected.

10. Method according to one of Claims 1 to 9, characterized in that for the physical structure disaggregation, the partially crosslinked polymerizate is subjected to a high-energy irradiation.

11. Method according to one of Claims 1 to 10, characterized in that for the physical structure disaggregation, the partially crosslinked polymerizate is subjected to a mechanical treatment leading to fissuring or to tension and deformation conditions in a surface layer.

12. Method according to Claim 11, characterized in that for the mechanical treatment, a ball mill, a high-speed stirrer, a jet pulverizer, a vibratory mill, a roll mill, an edge mill, a disk attrition mill or a cutting mill is used.

13. Use of the partially crosslinked polymerizate, produced according to one of Claims 1 to 12, with increased swelling rate for the production of a dental material.

0032124

14. Use of the partially crosslinked polymerizate, produced according to one of Claims 1 to 12, with increased swelling rate for the fabrication of crowns, bridges, veneers, and artificial teeth as well as fillings and denture material.